## Europäisches Patentamt

## European Patent Office

## Office européen des brevets

(19)

(11) Veröffentlichungsnummer: **0 357 024**
**A2**

(12) # EUROPÄISCHE PATENTANMELDUNG

(21) Anmeldenummer: **89115992.3**

(22) Anmeldetag: **30.08.89**

(51) Int. Cl.5: **C12N 15/31 , C12Q 1/68 , G01N 33/569 , A61K 39/40 , A61K 39/104 , //(C12N15/31, C12R1:385)**

Patentansprüche für folgende Vertragsstaaten:
ES + GR.

(30) Priorität: **01.09.88 DE 3829616**

(43) Veröffentlichungstag der Anmeldung:
**07.03.90 Patentblatt 90/10**

(84) Benannte Vertragsstaaten:
**AT BE CH DE ES FR GB GR IT LI LU NL SE**

(71) Anmelder: **BEHRINGWERKE Aktiengesellschaft**
**Postfach 1140**
**D-3550 Marburg 1(DE)**

(72) Erfinder: **Duchene, Michael, Dr.**
**Gabelsbergerstrasse 59**
**D-8000 München 2(DE)**
Erfinder: **von Specht, Ulrich, Prof.-Dr.**
**Am Waldweg**
**D-8193 Ambach(DE)**
Erfinder: **Domdey, Horst, Dr.**
**Fasanenweg 6**
**D-8027 Neuried(DE)**

(74) Vertreter: **Becker, Heinrich Karl Engelbert, Dr. et al**
**HOECHST AKTIENGESELLSCHAFT Central**
**Patent Department P.O. Box 80 03 20**
**D-6230 Frankfurt am Main 80(DE)**

(54) **Lipoprotein I (OMPI) von Pseudomonas aeruginosa.**

(57) Das Gen für das äußere Membranprotein Lipoprotein I (OMPI) von Pseudomonas aeruginosa ATCC 33354 konnte sequenziert und die Aminosäuresequenz abgeleitet werden. Hierdurch kann dieses Protein und immunogene Teilsequenzen hiervon in der Menge und Reinheit gewonnen werden, die für einen Einsatz zur Herstellung von Impfstoffen erforderlich sind.

EP 0 357 024 A2

## Lipoprotein I (OMPI) von Pseudomonas aeruginosa

Pseudomonas aeruginosa ist ein ubiquitär vorkommender Mikroorganismus, der in der Humanmedizin als "Problemkeim" gilt. Er befällt in erster Linie geschwächte Patienten und ist häufig durch Antibiotikatherapie nur schwer zu bekämpfen. Besonders gefährdet sind Patienten auf Intensivstationen und Querschnittsgelähmte sowie Menschen, die Verbrennungen erlitten haben oder einer erhöhten Gefahr von Verbrennungen ausgesetzt sind, wie Feuerwehrleute oder Stahlarbeiter. In all diesen Fällen besteht eine Aussicht, durch aktive Immunisierung einer Infektion vorzubeugen. Das Lipoprotein I (OMPI) ist wie Porin F (OMPF) ein Hauptbestandteil der äußeren Membran von P. aeruginosa und deswegen ebenfalls potentiell als Bestandteil einer Vakzine geeignet.

Der Erfindung liegt die komplette Charakterisierung des Gens für OMPI aus P. aeruginosa, Serotyp 6, ATCC 33354, zugrunde. Die DNA-Sequenz und die daraus abgeleitete Aminosäuresequenz sind in der Tabelle wiedergegeben, wobei die Aminosäuresequenz im Dreibuchstabencode unter den betreffenden Basentripletts angeordnet ist und das Signalpeptid durch kursive Buchstaben hervorgehoben ist.

Hierzu wurden aus P. aeruginosa die Proteine der äußeren Membran gewonnen und daraus nach klassischen Methoden monoklonale Antikörper gewonnen.

Aus dem Stamm wurde genomische DNA isoliert, gereinigt und eine Lambda EMBL3 Genbank wurde angelegt wie beschrieben (A.-M. Frischauf et al., J. Mol. Biol. 170 (1983) 827-842; M. Duchêne et al., J. Bacteriol. 170 (1988) 155-162). Diese Genbank wurde ausplattiert und Material aus den erhaltenen Plaques wurde auf Nitrocellulosemembranen transferiert. Mit Hilfe eines monoklonalen Antikörpers, der spezifisch für das Lipoprotein I von P. aeruginosa ist, wurde der Plaque detektiert, der geringe Mengen von Lipoprotein I exprimierte. Hierbei wurde die Antikörper-Antigenreaktion mit Hilfe eines zweiten, mit alkalischer Phosphatase gekoppelten Antikörpers und einer passenden Farbreaktion sichtbar gemacht. Das für Lipoprotein I kodierende Gen befand sich auf einem 15 kb großen DNA-Abschnitt, den der positive Lambda-Phage enthielt und konnte im folgenden auf ein 626 bp großes TaqI Fragment eingegrenzt werden. Beide Stränge der DNA wurden vollständig nach dem Prinzip von Sanger (Sanger et al., Proc. Natl. Acad. Sci. USA 74 (1977) 5463-5467) sequenziert. Aus der so erhaltenen DNA-Sequenz wurde die entsprechende Aminosäuresequenz abgeleitet. Der N-Terminus des reifen Proteins wurde aus dem Vergleich mit dem entsprechenden E.coli Protein abgeleitet, bei dem das reife Protein ebenfalls mit einem Cysteinrest beginnt, der sich im identischen Sequenzzusammenhang Gly-Cys-Ser-Ser (K. Nakamura und M. Inouye, Cell 18 (1979) 1109-1117) befindet wie bei P. aeruginosa.

Die Isolierung des Gens erlaubt nunmehr die Herstellung von Lipoprotein I und immunogenen Teilsequenzen dieses Proteins in der Menge und Reinheit, die für einen Einsatz zur Herstellung von Impfstoffen erforderlich ist.

Die Erfindung betrifft somit das Lipoprotein I (OMPI) mit der Aminosäuresequenz gemäß Tabelle, die dafür kodierende DNA, deren proteinkodierender Strang in der Tabelle dargestellt ist, immunogene Teilsequenzen von Lipopro tein I, mit Lipoprotein I und immunogenen Teilsequenzen dieses Proteins gewonnene polyklonale und monoklonale Antikörper und die entsprechenden Seren sowie Diagnostika, die solche Antikörper oder entsprechende Nukleotidsequenzen enthalten und Diagnostizierverfahren unter Verwendung solcher Diagnostika sowie gentechnische Verfahren zur Herstellung von OMPI oder immunogenen Teilproteinen.

Darüberhinaus eröffnet die Erfindung einen Weg zur passiven Immunisierung mit humanen monoklonalen Antikörpern. Die Erfindung betrifft deshalb auch die Verwendung von erfindungsgemäß gewonnenen Antigenen zur Induzierung von Lymphozyten zur Produktion entsprechender monoklonalen Antikörper bzw. zum Testen von Lymphozyten auf die Produktion solcher Antikörper.

Die Aufarbeitung, Reinigung, Immunisierung und Gewinnung der Seren bzw. Antikörper kann nach an sich bekannten Methoden erfolgen. Verwiesen wird beispielsweise auf M.E. Gilleland et al., Infection and Immunity 44 (1984) 49-54; R.E.W. Hancock et al., J. Infectious Diseases 149 (1984) 220-226; S. Sawada et al., J. Infectious Diseases 150 (1984) 570-576.

Die Erfindung wird in den folgenden Beispielen näher erläutert. Teile und Prozentangaben beziehen sich auf das Gewicht, wenn keine anderen Angaben gemacht sind.

Beispiel 1 -

Gewinnung von Lipoprotein I:

2

Die Gewinnung des Lipoprotein I hält sich an die Vorschrift von Inouye et al. (J. of Bacteriology 127 (1976) 555-563).

## Beispiel 2

Gewinnung von monoklonalen Antikörpern gegen Lipoprotein I:

Gereinigtes Lipoprotein I wird in Freunds Adjuvans oder Al(OH)$_3$ Balb/c Mäusen intraperitoneal injiziert. Nach 6 Wochen wird mit gelöstem Antigen "geboostet". Der Antikörpertiter wird eine Woche später durch ELISA-Tests bestimmt. Bei unzureichender Immunreaktion erfolgen weitere Injektionen. 3 Tage vor der Zellfusion werden die Mäuse mit 10 $\mu$g des gelösten Antigens intravenös "geboostet". Die Milzzellen werden nach Standardmethoden (nach G. Köhler und C. Milstein, Nature 256 (1975) 495-497) mit NS1-Zellen fusioniert. Nach Selektion in HAT-Medium wachsen in den Mikronäpfen einzelne Kolonien aus, deren Kulturüberstände wiederum im ELISA auf Antikörper gegen Lipoprotein I getestet werden. Positive Kolonien werden subkloniert. Die Antikörper werden aus Kulturüberständen und aus in Balb/c-Mäusen induzierten Asciten gewonnen, nach gängigen biochemischen Methoden gereinigt und charakterisiert.

## Beispiel 3

Gewinnung und Charakterisierung der Lipoprotein I DNA

Konstruktion einer Genbank von P. aeruginosa:

Die Konstruktion der Genbank ist publiziert (M. Duchêne et al. J. Bacteriol. 170 (1988) 155-162) und wurde entsprechend durchgeführt.

"Screenen" der Genbank nach Lipoprotein I Sequenzen:

Rekombinante Phagen werden in einer Dichte von 500 pfu * auf einem NM539 Bakterienrasen ausplattiert und über Nacht inkubiert. Die Phagenplaques werden 6 Stunden bei 37° C weiterinkubiert und so auf Nitrocellulosemembranen übertragen. Die positiven Plaques werden durch ihren Gehalt von Lipoprotein I identifiziert, indem die Filter zuerst mit dem monoklonalen Antikörper 6A4 inkubiert werden und daraufhin die positiven Antikörper-Antigenreaktion mit Hilfe eines zweiten Antikörpers und einer enzymatischen Farbreaktion mit alkalischer Phosphatase identifiziert wird.

Sequenzanalyse des Lipoprotein I Gens und seiner flankierenden Bereiche:

Von einem der isolierten Phagen wird eine Großkultur (1l) gezüchtet und daraus DNA präpariert (Maniatis et al., Molecular Cloning, Cold Spring Harbor Publications, 1982). Diese wird mit dem Restriktionsenzym Sall gespalten und die erhaltenen Restriktionsfragmente werden "shotgun" in pBR322 (Bolivar et al., Gene 2 (1977) 95-113) und später in pUC19 (Yanisch-Perron et al., Gene 33 (1985) 103-119) subkloniert. Dabei wird das Lipoprotein I (OMPI) in den transformierten E.coli Zellen ebenfalls in ausreichender Menge exprimiert, so daß positive Transformanten mit einer Antikörperreaktion erkannt werden können. Der kleinste Subklon, der das Protein exprimiert, ist der Klon pITaq. Aus diesem Klon wird Plasmid-DNA isoliert und diese nach ExoIII und ExoVII Verdauung (Yanisch-Perron et al., s.o.) nach der Methode von Chen und Seeburg (DNA 4 (1985) 165-170) sequenziert. Der Klon besitzt ein 626 bp großes TaqI Insert, das die vollständige für das Lipoprotein I (OMPI) kodierende Sequenz enthält. Diese Sequenz ist in der Tabelle dargestellt.

* pfu = plaque forming units

3

Beispiel 4

Expression von Lipoprotein I:

Da der Promoter, der in P. aeruginosa die Transkription des Lipoprotein I Gens treibt, dem E.coli Konsensus-Promoter sehr ähnlich ist, wird in allen bisher untersuchten Plasmiden, die die vollständige Sequenz der Lipoprotein I Transkriptionseinheit enthalten, das Protein exprimiert.

Das Lipoprotein I wird erhalten, indem man es aus einer Kultur von E.coli Zellen, die mit dem Plasmid pITaq transformiert sind, nach der oben zitierten Methode von Inouye et al. isoliert.

Beispiel 5

Herstellung von Antiseren:

Gesunde Erwachsene, die in ihrer Krankheitsgeschichte keine Allergien, Diabetes, Immundefizienz-Krankheiten, Anämien oder Hautkrankheiten aufweisen, werden mit heterolog exprimiertem Lipoprotein I, bzw. Teilsequenzen davon, immunisiert. Die Vaccinierung erfolgt an den Tagen 1, 8 und 15. Drei Wochen nach der letzten Injektion wird den freiwilligen Kandidaten Blut entnommen und dieses auf Hepatitis B Oberflächenantigen sowie auf HIV Antigene getestet. Nur negativ reagierende Plasmaspenden werden gepoolt, unter kontrollierten sterilen Bedingungen fraktioniert und abgepackt.

Tabelle

```
1                              20                              40
ATG AAC AAC GTT CTG AAA TTC TCT GCT CTG GCT CTG GCT GCT GTT
Met Asn Asn Val Leu Lys Phe Ser Ala Leu Ala Leu Ala Ala Val
                    60                              80
CTG GCC ACC GGT TGC AGC AGC CAC TCC AAA GAA ACC GAA GCT CGT
Leu Ala Thr Gly Cys Ser Ser His Ser Lys Glu Thr Glu Ala Arg
            100                             120
CTG ACC GCT ACC GAA GAC GCA GCT GCT CGT GCT CAG GCT CGC GCT
Leu Thr Ala Thr Glu Asp Ala Ala Ala Arg Ala Gln Ala Arg Ala
        140                     160                     180
GAC GAA GCC TAT CGC AAG GCT GAC GAA GCT CTG GGC GCT GCT CAG
Asp Glu Ala Tyr Arg Lys Ala Asp Glu Ala Leu Gly Ala Ala Gln
                            200                     220
AAA GCT CAG CAG ACC GCT GAC GAG GCT AAC GAG CGT GCC CTG CGC
Lys Ala Gln Gln Thr Ala Asp Glu Ala Asn Glu Arg Ala Leu Arg
                    240
ATG CTG GAA AAA GCC AGC CGC AAG TAA TAG
Met Leu Glu Lys Ala Ser Arg Lys *   *
```

Tabelle: Sequenz des Lipoprotein I von *Pseudomonas aeruginosa*. Der kodierende Bereich ist abgebildet mit den Aminosäureresten im Dreibuchstabencode. Das Signalpeptid ist kursiv gedruckt.

**Ansprüche**

1. Äußeres Membranprotein Lipoprotein I (OMPI) von Pseudomonas aeruginosa mit der Aminosäuresequenz gemäß Tabelle.

2. DNA, kodierend für das Lipoprotein I nach Anspruch 1.

3. Immunogene Teilsequenzen des Lipoprotein I (IMPI) nach Anspruch 1.

4. Verfahren zur Herstellung von OMPI oder immunogenen Teilsequenzen gemäß Tabelle, dadurch gekennzeichnet, daß die dafür kodierende DNA in pro- oder eukaryotischen Zellen zur Expression gebracht wird.

5. Polyklonale oder monoklonale Antikörper, erhalten unter Verwendung von Lipoprotein I gemäß Anspruch 1 oder immunogener Teilsequenzen dieses Proteins nach Anspruch 3 als Antigen.

6. Diagnostikum, enthaltend Antikörper nach Anspruch 5.

7. Diagnostikum, das die Nukleotidsequenz gemäß Anspruch 2 ganz oder teilweise oder eine davon abgeleitete Nukleotidsequenz enthält.

8. Diagnostizierverfahren unter Verwendung eines Diagnostikums nach Anspruch 6 oder 7.

9. Verwendung eines Proteins nach Anspruch 1 oder 3 zur Induzierung von Lymphozyten zur Produktion von Antikörpern.

10. Verwendung eines Proteins nach Anspruch 1 oder 3 zum Testen von Lymphozyten auf die Produktion von Antikörpern gegen solche Proteine.

Patentansprüche für folgende Vertragsstaaten:ES,GR

1. Verfahren zur Herstellung des Äußeren Membranproteins (Lipoproteins) I (OMPI) gemäß Tabelle oder immunogenen Teilsequenzen, dadurch gekennzeichnet, daß die dafür kodierende DNA in pro- oder eukaryotischen Zellen zur Expression gebracht wird.

2. Verfahren zur Herstellung von polyklonalen oder monoklonalen Antikörpern, dadurch gekennzeichnet, daß das OMPI gemäß Tabelle oder immunogenen Teilsequenzen davon zur Immunisierung eingesetzt werden.

3. Verfahren zum Nachweis von Pseudomonas aeruginosa Infektionen, dadurch gekennzeichnet, daß das OMPI gemäß Tabelle oder immunogene Teilsequenzen eingesetzt werden.

4. Verfahren zum Nachweis von Pseudomonas aeruginosa Infektionen, dadurch gekennzeichnet, daß die für OMPI gemäß Tabelle oder Teilsequenzen kodierende DNA oder RNA zur Hybridisierung eingesetzt werden.